(19) Europäisches Patentamt
European Patent Office
Office européen des brevets

(11) Veröffentlichungsnummer: **0 296 404 B1**

(12) **EUROPÄISCHE PATENTSCHRIFT**

(45) Veröffentlichungstag der Patentschrift: **16.09.92**

(51) Int. Cl.$^5$: **C07C 51/60**, C07C 53/42

(21) Anmeldenummer: **88109086.4**

(22) Anmeldetag: **08.06.88**

# (54) Verfahren zur Gewinnung von Carbonsäurehalogeniden mit mehr als 7 Kohlenstoffatomen.

(30) Priorität: **12.06.87 DE 3719640**

(43) Veröffentlichungstag der Anmeldung:
**28.12.88 Patentblatt 88/52**

(45) Bekanntmachung des Hinweises auf die Patenterteilung:
**16.09.92 Patentblatt 92/38**

(84) Benannte Vertragsstaaten:
**AT BE DE ES FR GB IT NL SE**

(56) Entgegenhaltungen:
**EP-A- 0 027 941**
**EP-A- 0 031 504**
**EP-A- 0 252 408**
**DD-A- 153 867**
**DD-A- 220 597**
**US-A- 1 936 739**
**US-A- 3 557 205**

(73) Patentinhaber: **BASF Aktiengesellschaft**
**Carl-Bosch-Strasse 38**
**W-6700 Ludwigshafen(DE)**

(72) Erfinder: **Freudenberg, Enrique, Dr.**
**Albert-Einstein-Allee 33**
**W-6703 Limburgerhof(DE)**
Erfinder: **Wittmer, Peter**
**Albrecht-Dürer-Ring 29 a**
**W-6710 Frankenthal(DE)**
Erfinder: **Hohmann, Andreas, Dr.**
**Sinsheimer Strasse 22**
**W-6700 Ludwigshafen(DE)**
Erfinder: **Bechtolsheimer, Hans-Heinrich, Dr.**
**Ringstrasse 7**
**W-6521 Dittelsheim-Hessloch(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft ein neues and verbessertes Verfahren zur Herstellung von Carbonsäurechloriden bzw. -bromiden mit mehr als 7 C-Atomen durch Umsetzung der entsprechenden Carbonsäuren mit Phosphorchloriden bzw. -bromiden.

Carbonsäurehalogenide sind wichtige Zwischenprodukte bei der Synthese einer Vielzahl chemischer Produkte, z.B. Pharmazeutika, Tenside oder Masseleimungsmittel für Papier. Von technischem Interesse sind insbesondere Carbonsäurechloride.

Die Herstellung von Carbonsäurechloriden durch Umsetzung von Carbonsäuren mit Phosphorchloriden wie Phosphorpentachlorid oder Phosphortrichlorid ist altbekannt, wobei die Umsetzung mit Phosphortrichlorid aus wirtschaftlichen Gründen vorteilhafter ist, da im Fall des Pentachlorids nur 20 % des Chlors zur Carbonsäurechloridbildung genutzt werden. Die anfallenden Säurechloride werden in der Regel durch Destillation gereinigt, wobei nicht umgesetzte Ausgangsstoffe wie Säure und Phosphortrichlorid und Nebenprodukte der Reaktion wie Carbonsäureanhydride, Phosphonocarbonsäureanhydride bzw. andere Anhydride aus Carbonsäure und phosphoriger Säure als Vorlauf bzw. als Destillationsrückstand vom Wertprodukt abgetrennt werden. Diese Verunreinigungen können die Weiterverarbeitbarkeit der Säurechloride in starkem Maße beeinträchtigen.

Eine solche Destillation ist bei längerkettigen Carbonsäurechloriden infolge der hohen Siedetemperatur dieser Produkte mit großem apparativen Aufwand und mit Verlusten infolge thermischer Zersetzung verbunden. Die Zersetzungsprodukte müssen überdies entsorgt werden.

Die DD-PS 01 53 867 beschreibt ein zweistufiges Verfahren zur Herstellung von Carbonsäurechloriden durch Umsetzung von Säuren, z.B. Fettsäuren mit Phosphortrichlorid oder Thionylchlorid und gegebenenfalls Chlorwasserstoff, wonach die Produkte in verbesserter Reinheit ohne destillative Reinigung erhalten werden.

Die dort geschilderten diskontinuierlichen Verfahren liefern aber bei Verweilzeiten von mindestens acht Tagen Produkte, die noch jeweils mehrere Prozente nicht umgesetzte Säure und Säureanhydrid als Nebenprodukt enthalten.

Die kontinuierlichen Verfahrensweisen liefern bessere Produkte, erfordern aber den Einsatz komplizierter und umfangreicher technischer Apparaturen, wie zweier hintereinandergeschalteter Kesselkaskaden mit nachgeschalteten Trenngefäßen.

Die schwedische Anmeldung Nr. 8 203 397 vom 01.06.82 beschreibt die Umsetzung von Carbonsäuren, insbesondere Palmitinisäure und Ethylhexansäure, zu Säurechloriden durch

- Umsetzen mit Phosphortrichlorid und
- Nachbehandeln des rohen Säurechlorids mit Phosgen oder Thionylchlorid.

Hierdurch werden ohne Destillation Säurechloride mit einem Gehalt an nicht umgesetzter Säure von 0,3 % oder weniger erhalten.

Nachteilig an dieser Verfahrensweise sind jedoch die mit dem Zweithalogenierungsmittel verbundenen Probleme wie etwa der erforderliche sicherheitstechnische Aufwand bei der Handhabung von Phosgen oder die Entsorgung des als Folgeprodukt bei Verwendung von Thionylchlorid entstehenden Schwefeldioxids. Zudem besteht die Gefahr der Kontamination des Carbonsäurechlorids mit dunkelgefärbten und übelriechenden schwefelorganischen Verunreinigungen.

Der vorliegenden Erfindung lag daher die Aufgabe zugrunde, einen besseren Zugang zu den Carbonsäurechloriden bzw. -bromiden mit mehr als 7 C-Atomen zu finden und den Nachteilenh der bekannten Verfahren abzuhelfen.

Demgemäß wurde ein neues und verbessertes Verfahren zur Gewinnung von Carbonsäurehalogeniden der allgemeinen Formel I

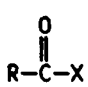

(I),

in der R einen organischen Rest mit mehr als 7 C-Atomen und X Chlor oder Brom bedeutet, durch Umsetzung von Carbonsäuren der allgemeinen Formel II

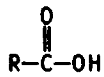

(II),

in der R die oben genannte Bedeutung hat, mit Phosphorchloriden bzw. Phosphorbromiden gefunden, welches dadurch gekennzeichnet ist, daß man diese Reaktionsgemische mit Carbonsäureamid-Hydrochlorid oder Hydrobromid Gemischen behandelt, die mit den Carbonsäurehalogeniden I nich homogen mischbar sind, und die Carbonsäureamid Hydrochlorid oder -Hydrobromid-Phase danach abtrennt.

Wesentlich für den Erfolg des erfindungsgemäßen Verfahren ist, daß das verwendete Carbonsäureamid-Hydrochlorid bzw. -Hydrobromid mit dem entstehenden Carbonsäurehalogenid I nicht homogen mischbar ist, wodurch das Endprodukt durch einfache Phasentrennung des nach der Behandlung anfallenden Reaktionsgemisches abgetrennt werden kann. Weiterhin ermöglicht die zweiphasige Arbeitsweise die einfache Rückführung der Carbonsäureamidhydrohalogenidphase für weitere Nachbehandlungen.

Abgesehen von der nicht homogenen Mischbarkeit mit dem Produkt, bestehen hinsichtlich der Art des Carbonsäureamid-Hydrochlorids bzw. -Hydrobromids keinerlei Einschränkungen. Zweckmäßigerweise wird man bei der Herstellung von Carbonsäurechloriden bzw. -bromiden, Carbonsäureamid-Hydrochloride bzw. -Hydrobromide verwenden, die z.B. in situ aus dem Carbonsäureamid und Chlorwasserstoff bzw. Bromwasserstoff herstellbar sind. Geeignete Carbonsäureamide sind solche von niedermolekularen Carbonsäuren wie Buttersäure, Propionsäure, Essigsäure oder Ameisensäure und primären oder sekundären Aminen wie Alkylaminen, Dialkylaminen, N-Methylanilin oder Piperidin. Bevorzugte Säureamide sind solche der allgemeinen Formel III

$R^1$-CO-N$R^2R^3$ (III),

in der $R^1$ Wasserstoff oder einen Methyl-, Ethyl-, Propyl, Isopropyl- oder Butylrest darstellt und $R^2$ und $R^3$ für Wasserstoff, $C_1$-$C_4$-Alkyl, $C_5$- oder $C_6$-Cycloalkyl, Benzyl oder Phenyl stehen oder beide Reste $R^2$ und $R^3$ miteinander zu einer $C_4$- oder $C_5$-Alkylenbrücke verknüpft sind, d.h. zusammen mit dem Stickstoff einen 5- oder 6-gliedrigen Ring bilden.

Für die Gewinnung von Carbonsäurechloriden bzw. -bromiden I mit mehr als 7 C-Atomen können besonders vorteilhaft N,N-Dialkylformamid-Hydrochloride verwendet werden, die weniger als 8 Kohlenstoffatome aufweisen. Beispielsweise seien N,N-Dimethyl-, N,N-Diethyl-, N-Methyl-, N-Ethyl-, N,N-Dipropyl oder N,N-Diisopropylformamid aufgeführt, wobei wegen der leichten Zugänglichkeit N,N-Dimethylformamid besonders bevorzugt ist.

Im allgemeinen sind katalytische Mengen des Carbonsäureamids, bezogen auf eingesetzte Carbonsäure, ausreichend. Beispielsweise können Mengen von 0,5 bis 100 mol-%, insbesondere 2 bis 20 mol-%, bezogen auf Carbonsäure II verwendet werden. Größere Mengen sind möglich, bringen aber i.a. keine weiteren Vorteile.

Die Nachbehandlung am Beispiel der Carbonsäurechloride erfolgt zweckmäßig in der Weise, daß man das Carbonsäureamid dem nach der Chlorierung anfallenden Reaktionsgemisch zugibt und anschließend Salzsäure gasförmig oder flüssig hinzufügt. Die Salzsäure kann, bezogen auf das N,N-Dialkylcarbonsäureamid in Molverhältnissen von 0,5 zu 1 bis 3 zu 1, bevorzugt im Molverhältnis 1 zu 1 bis 2 zu 1, eingesetzt werden. Das sich hierbei bilden zweiphasige Gemisch wird gut durchgerührt und am Ende die beiden Phasen getrennt. Analog kann man bei den Carbonsäurebromiden verfahren. Die Temperatur liegt in der Regel in dem Bereich, in dem die Chlorierung bzw. Bromierung vorgenommen wurde, z.B. bei 20 bis 70, insbesondere 30 bis 60 °C.

Die Halogenierung der Carbonsäuren erfolgt in an sich bekannter Weise, so daß sich nähere Ausführungen hierzu erübrigen.

Der Ausdruck Carbonsäure wird hier für sämtliche Säuren verwendet, die gemäß allgemeiner Definition normalerweise Säurehalogenide bei der Reaktion mit phosphorhaltigen Chlorierungs- bzw. Bromierungsmitteln wie Phosphorchloriden, beispielsweise Phosphorpentachlorid und Phosphortrichlorid bzw. Phosphorbromiden, beispielsweise Phosphortribromid bilden. Das Verfahren gemäß der Erfindung kann z.B. bei der Chlorierung bzw. Bromierung gewöhnlicher aliphatischer Carbonsäuren verwendet werden, die höher- oder niedermolekulare aliphatische Gruppen enthalten, bei Monoestern von Dicarbonsäuren, Ketonsäuren usw. Vorzugsweise eignet sich das Verfahren gemäß der Erfindung zur Herstellung von Säurehalogeniden aus aliphatischen Carbonsäuren, und zwar vor allem aus Monocarbonsäuren, d.h. zur Herstellung von Verbindungen mit der allgemeinen Formel I

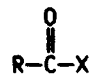

(I),

wobei R eine aliphatische Kohlenwasserstoffgruppe darstellt. Die aliphatische Gruppe kann geradkettig oder verzweigt, gesättigt, olefinisch oder acetylenisch ungesättigt sein. Besonders bevorzugt sind aliphatische Carbonsäuren mit 8 bis 28, insbesondere 12 bis 22 Kohlenstoffatomen.

Die Umsetzung der Carbonsäure II, beispielsweise mit Phosphortrichlorid, kann man vorteilhaft so durchführen, daß man bei Temperaturen von 20 bis 70 ° C, bevorzugt bei Temperaturen von 40 bis 60 ° C, die Carbonsäure oder das Phosphortrichlorid gegebenenfalls in Gegenwart eines inerten Lösungsmittels vorlegt und die Säure oder Phosphortrichlorid, gegebenenfalls mit dem gleichen inerten Lösungsmittel verdünnt, zugibt. Auch eine gleichzeitige Zugabe von Carbonsäure und Chlorierungsmittel ist möglich. Phosphortrichlorid kann in Mengenverhältnissen von einem mol Carbonsäure zu 0,4 mol Phosphortrichlorid bis zu einem mol Carbonsäure zu 0,8 mol Phosphortrichlorid eingesetzt werden, wobei ein Verhältnis von 1:0,5 bis 1:0,6 bevorzugt ist.

Als inerte Lösungsmittel können Kohlenwasserstoffe, halogenierte Kohlenwasserstoffe, z.B. Chlorkohlenwasserstoffe oder Ether Verwendung finden; bevorzugt sind aromatische Kohlenwasserstoffe wie Toluol oder Xylole.

Nach beendeter Zugabe von Carbonsäure II oder Phosphortrichlorid, Absitzenlassen und Abtrennen der gemäß folgender Reaktionsgleichung entstandenen phosphorigen Säure

$$3\ R\text{-}COOH + PCl_3 \rightarrow 3\ R\text{-}COCl + H_3PO_3$$

kann das Reaktionsgemisch unter ständigem, gelegentlichem oder auch ohne Rühren einer Nachreaktion unterworfen werden. Hierbei kann am Ende oder auch mehrfach zwischenzeitlich, ausgefallene phosphorige Säure, die als Wertprodukt für andere Synthesen eingesetzt werden kann, abgetrennt werden.

Die weitere Aufarbeitung nach der erfindungsgemäßen Nachbehandlung erfolgt in der Weise, daß man die zwei flüssigen Phasen trennt, wobei man die Carbonsäureamid-Hydrochlorid bzw. -Hydrobromid Phase ggf. nach Zugabe von Chlorwasserstoff bzw. Bromwasserstoff vorteilhaft für weitere Nachbehandlungen wiederverwenden kann und aus der Carbonsäurehalogenid-haltigen Phase flüchtige Bestandteile wie überschüssiges Phosphorhalogenid und ggf. Lösungsmittel in an sich bekannter Weise z.B. destillativ bei Normaldruck oder leichtem Unterdruck entfernt, wobei die Temperatur unterhalb der Zersetzungstemperatur des Carbonsäurehalogenids I, vorteilhaft unter ca. 75 ° C, liegen sollte.

Das zurückgewonnene Lösungsmittel-Phosphorhalogenid-Gemisch kann für neue Umsetzungen wiederverwendet werden. Das zurückbleibende schwerflüchtige Carbonsäurehalogenid kann Restmengen des inerten Lösungsmittels von 2 bis 5 % enthalten, die insbesondere bei der Weiterverarbeitung zu Folgeprodukten unter Verwendung des gleichen Lösungsmittels nicht stören und gegebenenfalls auf einer Folgestufe zurückgewonnen werden können.

Nach dem erfindungsgemäßen Verfahren können Carbonsäurehalogenide in hoher Reinheit mit Gehalten an nicht umgesetzter Carbonsäure unter 0,1 %, Carbonsäureanhydridgehalten unter 1 % und nur geringen Gehalten an phoshorhaltigen Verunreinigungen erhalten werden.

Im folgenden soll die Erfindung beispielhaft erläutert werden; die angegebenen Teile und Prozente beziehen sich auf das Gewicht, sofern nichts anderes angegeben ist.

Beispiel 1 (Vergleichsbeispiel)

In einem mit Rührer, Heizung und Bodenablaß ausgestattetem Reaktionsgefäß werden unter Feuchtigkeitsausschluß 275 Teile Phosphortrichlorid und 300 Teile wasserfreies Toluol vorgelegt und auf 40 ° C erwärmt. Während 5 Stunden werden dann unter Halten der Temperatur zwischen 40 und 45 ° C und Rühren 1100 Teile aufgeschmolzene technische Stearinsäure zugegeben. Nach kurzem Absitzen wird die abgeschiedene phosphorige Säure abgetrennt und das Reaktionsgemisch noch 2 Stunden bei 40 ° C nachgerührt. Nach kurzem Absitzen wird nochmals ausgefallene phosphorige Säure abgetrennt.

Unumgesetztes Phosphortrichlorid und überschüssiges Lösungsmittel werden bei maximal 75 ° C Produkttemperatur und 25 mbar Vakuum abdestilliert, bis nichts mehr übergeht. Für dieses Stearinsäurechlorid, das noch 1,5 % Toluol enthielt, wurden folgende analytische Daten (berechnet auf 100 %iges, toluolfreies Produkt) erhalten:

90,1 % Stearinsäurechlorid, <1 % Stearinsäure, 4,1 % Stearinsäureanhydrid, <0,05 % Phosphortrichlorid,

370 ppm Phosphor.

Beispiel 2 (Vergleichsbeispiel)

Gemäß Beispiel 1 wird eine toluolische Stearinsäurechloridlösung erzeugt. Vor der abschließenden Destillation werden, bezogen auf die eingesetzte Stearinsäure, 20 mol-% Salzsäure gasförmig bei einer Temperatur von 40 bis 45 °C unter Rühren während einer Stunde ins Reaktionsgemisch eingeleitet. Anschließend werden, wie in Beispiel 1 beschrieben, die flüchtigen Anteile abdestilliert.

Für dieses Säurechlorid, das noch 2,4 % Toluol enthielt, wurden folgende analytische Daten (berechnet auf 100 %iges, toluolfreies Produkt) erhalten:
91,3 % Stearinsäurechlorid, <1 % Stearinsäure, 4,1 % Stearinsäureanhydrid, <0,05 % Phosphortrichlorid, 600 ppm Phosphor.

Beispiel 3

Gemäß Beispiel 1 wird eine toluolische Stearinsäurechloridlösung erzeugt. Vor der abschließenden Destillation werden 10 mol-% Dimethylformamid zugegeben und bei einer Temperatur von 40 bis 45 °C 20 mol-% Salzsäure (mol-%-Angaben jeweils bezogen auf die eingesetzte Stearinsäure) gasförmig innerhalb einer Stunde unter Rühren ins Reaktionsgemisch geleitet. Hierbei bildet sich eine zweite, mit der toluolischen Stearinsäurechloridlösung nicht mischbare Phase. Das zweiphasige Gemisch wird zwei Stunden bei 40 bis 45 °C kräftig durchgerührt und die Dimethylformamidhydrochloridphase dann abgetrennt. Anschließend werden, wie in Beispiel 1 geschildert, die flüchtigen Anteile abdestilliert. Für diese Säurechlorid, das noch 2,6 % Toluol enthielt, wurden folgende analytische Daten (berechnet auf 100 %iges, toluolfreies Produkt) erhalten:
96,0 % Stearinsäurechlorid, <0,1 % Stearinsäure, <1,0 % Stearinsäureanhydrid, <0,05 % Phosphortrichlorid, 1400 ppm Phosphor, 150 ppm Gesamtstickstoff.

Beispiel 4

Gemäß Beispiel 1 wird eine toluolische Stearinsäurechloridlösung erzeugt. Vor dem abschließenden Abdestillieren der flüchtigen Anteile wird die Dimethylformamidhydrochloridphase aus Beispiel 3 zugegeben und anschießend werden, bezogen auf die eingesetzte Stearinsäure, 20 mol-% Salzsäure gasförmig bei 40 bis 45 °C in einer Stunde eingeleitet. Die zweiphasige Mischung wird bei dieser Temperatur kräftig gerührt und die Dimethylformamidhydrochloridphase dann abgetrennt. Anschließend werden, wie in Beispiel 1 geschildert, die flüchtigen Anteile abdestilliert. Für dieses Säurechlorid, das noch 2,7 % Tolouol enthielt, wurden folgende analytische Daten (berechnet auf 100 %iges, toluolfreies Säurechlorid) erhalten:
94,6 % Stearinsäurechlorid, <0,1 % Stearinsäure, <1,0 % Stearinsäureanhydrid, <0,05 % Phosphortrichlorid, 630 ppm Phosphor.

## Patentansprüche

**1.** Verfahren zur Gewinnung von Carbonsäurehalogeniden der allgemeinen Formel I

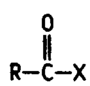
(I),

in der R einen organischen Rest mit mehr als 7 C-Atomen und X Chlor oder Brom bedeutet, durch Umsetzung von Carbonsäuren der allgemeinen Formel II

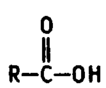
(II),

in der R die oben genannte Bedeutung hat, mit Phosphorchloriden bzw. Phosphorbromiden, dadurch gekennzeichnet, daß man diese Reaktionsgemische mit Carbonsäureamid-Hydrochlorid oder -

Hydrobromid Gemischen behandelt, die mit den Carbonsäurehalogeniden I nicht homogen mischbar sind, und die Carbonsäureamid-Hydrochlorid oder -Hydrobromid Phase danach abtrennt.

2. Verfahren nach Anspruch 1, dadurch gekennzeichnet, daß man die Reaktionsgemische mit 0,5 bis 100 mol-% Carbonsäureamid-Hydrochlorid bzw. -Hydrobromid Gemisch, bezogen auf die eingesetzte Carbonsäure II, behandelt.

3. Verfahren nach den Ansprüchen 1 bis 2, dadurch gekennzeichnet, daß man die Reaktionsgemische mit Carbonsäureamid-Hydrochlorid-Gemischen behandelt.

4. Verfahren nach den Ansprüchen 1 bis 2, dadurch gekennzeichnet, daß man die Reaktionsgemische mit Formamid-Chlorwasserstoff behandelt.

5. Verfahren nach den Ansprüchen 1 bis 4, dadurch gekennzeichnet, daß man Verbindungen der Formel II verwendet, in denen R für einen aliphatischen Rest mit 8 bis 28 Kohlenstoffatome steht.

6. Verfahren nach den Ansprüchen 1 bis 5, dadurch gekennzeichnet, daß man als Phosphorchlorid Phosphortrichlorid verwendet.

## Claims

1. A process for preparing a carbonyl halide of the formula I

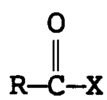

(I)

where R is an organic radical of more than 7 carbon atoms and X is chlorine or bromine, by reacting a carboxylic acid of the formula II

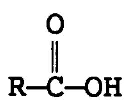

(II)

where R has the abovementioned meaning, with a phosphorus chloride or bromide, which comprises treating this reaction mixture with a carboxamide-hydrochloride or -hydrobromide mixture which is not homogeneously miscible with the carbonyl halide I, and then separating off the carboxamide hydrochloride or hydrobromide phase.

2. A process as claimed in claim 1, wherein the reaction mixture is treated with from 0.5 to 100 mol-% of a carboxamide-hydrochloride or -hydrobromide mixture, based on starting carboxylic acid II.

3. A process as claimed in claim 1 or 2, wherein the reaction mixture is treated with a carboxamide-hydrochloride mixture.

4. A process as claimed in claim 1 or 2, wherein the reaction mixture is treated with formamide-hydrogen chloride.

5. A process as claimed in any of claims 1 to 4, wherein in the compound of the formula II R is an aliphatic radical of from 8 to 28 carbon atoms.

6. A process as claimed in any of claims 1 to 5, wherein the phosphorus chloride used is phosphorus trichloride.

## Revendications

**1.** Procédé pour l'obtention d'halogénures d'acides carboxyliques de la formule générale I

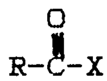

(I),

dans laquelle R désigne un reste organique contenant plus de 7 atomes de carbone et X désigne du chlore ou du brome, par réaction d'acides carboxyliques de la formule générale II

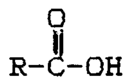

(II),

dans laquelle R a la signification sus-indiquée, avec des chlorures de phosphore ou des bromures de phosphore, caractérisé en ce que l'on traite ces mélanges réactionnels avec des mélanges amide d'acide carboxylique-chlorhydrate ou -bromhydrate qui ne sont pas miscibles de façon homogène avec les halogénures d'acides carboxyliques I, et en ce que l'on sépare ensuite la phase amide d'acide carboxylique-chlorhydrate ou -bromhydrate.

**2.** Procédé suivant la revendication 1, caractérisé en ce que l'on traite les mélanges réactionnels avec 0,5 à 100 % en moles du mélange amide d'acide carboxylique-chlorhydrate ou -bromydrate, par rapport à l'acide carboxylique II mis en oeuvre.

**3.** Procédé suivant la revendication 1 ou 2, caractérisé en ce que l'on traite les mélanges réactionnels avec des mélanges amide d'acide carboxylique-chlorhydrate.

**4.** Procédé suivant la revendication 1 ou 2, caractérisé en ce que l'on traite les mélanges réactionnels avec le mélange formamide-gaz chlorhydrique.

**5.** Procédé suivant l'une quelconque des revendications 1 à 4, caractérisé en ce que l'on utilise des composés de la formule II, dans laquelle R est mis pour un reste aliphatique contenant 8 à 28 atomes de carbone.

**6.** Procédé suivant l'une quelconque des revendications 1 à 5, caractérisé en ce que l'on utilise, comme chlorure de phosphore, du trichlorure de phosphore.